(19) Europäisches Patentamt
European Patent Office
Office européen des brevets

(11) Numéro de publication: **0 203 615**
**A1**

(12) # DEMANDE DE BREVET EUROPEEN

(21) Numéro de dépôt: **86107380.7**

(22) Date de dépôt: **30.05.86**

(51) Int. Cl.[4]: **C 07 C 11/21**, C 07 C 1/30

(30) Priorité: **31.05.85 FR 8508181**

(43) Date de publication de la demande: **03.12.86 Bulletin 86/49**

(84) Etats contractants désignés: **AT BE CH DE FR GB IT LI LU NL SE**

(71) Demandeur: **Oril S.A., 13 rue Auguste-Desgenétais, F-76210 Bolbec (FR)**

(72) Inventeur: **Fugier, Claude, 34 rue André Caplet, F-76600 Le Havre (FR)**
Inventeur: **Leroux, Michel, Nointot, F-76210 Bolbec (FR)**
Inventeur: **Normant, Jean-Francois, 9 rue Paul Doumer, F-92340 Bourg La Reine (FR)**
Inventeur: **Alexakis, Alexandre, 11 rue Charbonnel, F-75013 Paris (FR)**

(74) Mandataire: **Patentanwälte TER MEER - MÜLLER - STEINMEISTER, Mauerkircherstrasse 45, D-8000 München 80 (DE)**

(54) **Nouveau procédé de préparation des undécatriènes-1,3,5.**

(57) Procédé de préparation des undécatriènes (1, 3, 5) caractérisé en ce que l'on fait réagir un composé organométallique (5) d'heptène (1) yle avec un dérivé de butadiène (1E, 3).

Production des undécatriènes (1, 3, 5) utilisables à l'Industrie des parfums et arômes.

EP 0 203 615 A1

La présente invention concerne un nouveau procédé de synthèse de l'undécatriène-1,3,5 et plus précisément des isomères 3E,5Z (I) et 3E,5E (II) de cette polyoléfine.

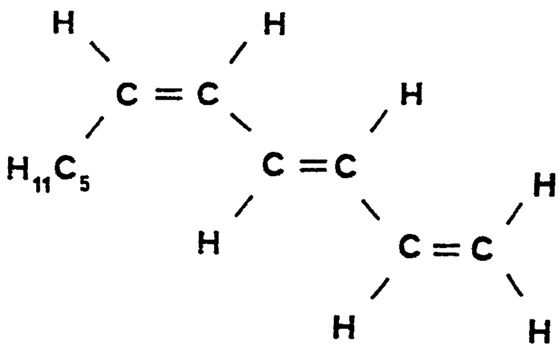

**Undécatriène-1,3E,5Z**
(I)

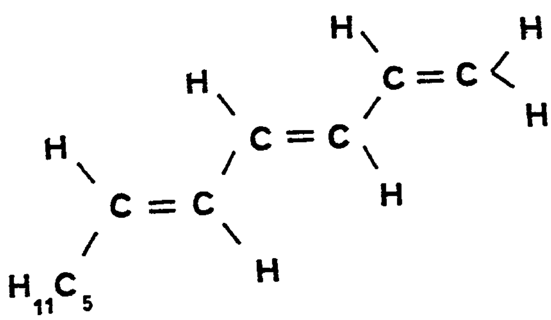

**Undécatriène-1,3E,5E**
(II)

Ces composés ont été isolés de certaines huiles essentielles et ont été utilisés pour leurs propriétés aromatisantes et odoriférantes.

La préparation industrielle de ces isomères dans leur stéréochimie requise pose de nombreux problèmes qui n'ont pas encore été résolus de manière satisfaisante.

La première synthèse proposée (Recherches 16,5-38, 1967) nécessite de nombreuses étapes aux rendements incompatibles avec les impératifs industriels.

D'autre part, l'application des procédés de la demande du brevet français N° 2.309.498 nécessite soit la longue et délicate préparation d'un halogénure de pentadiényl triphényl-phosphonium, soit l'utilisation du butène-1 époxyde-3,4 dont on connait par ailleurs les propriétés cancérigènes.

Les méthodes plus récentes n'apportent pas d'avantage puisqu'elles nécessitent soit la préparation difficile d'une sulfone particulièrement instable (Tetrahedron Letters 23(48), 5053-5046,1982), soit la semi-réduction, difficilement maîtrisable au stade industriel, d'une liaison triple conjuguée (Tetrahedron Letters 24(5),489-492,1983), soit encore la préparation à très basse température d'un dérivé lithien instable (Tetrahedron Letters 24(6),2665-2668,1983), ou la décyclisation des perhydrothiophènes polycycliques à très haute température (Tetrahedron Letters 26(10),1301-1304,1985).

La demanderesse a maintenant découvert un nouveau procédé de préparation de l'undécatriène-1,3E,5Z et de son isomère 3E,5E, très stéréoselectif et particulièrement simple et avantageux en regard des procédés connus.

L'invention a plus précisément pour objet un procédé de préparation de l'undécatriène-1,3E,5Z pur (ou en mélange avec l'undécatriène-1,3E,5E) caractérisé en ce qu'on fait réagir un composé organométallique d'heptène-1Z yle pur (ou en mélange avec le composé organométallique correspondant à l'isomère 1E), de formule :

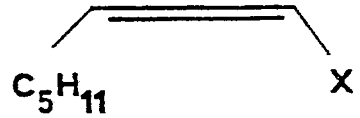

(III)

dans laquelle le symbole X représente un groupe partant choisi parmi les métaux de transition ou les halogènures de magnésium avec un dérivé de butadiène-1E,3 de formule :

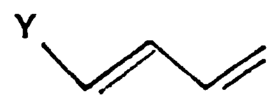

(IV)

dans laquelle le symbole Y représente un groupe partant choisi parmi les atomes d'halogène ou les radicaux alkylthio (comportant de 1 à 4 atomes de carbone) en présence d'un catalyseur métallique, dans un solvant organique inerte, à la température ambiante ou à une température légérement inférieure ou supérieure, puis on hydrolyse le milieu réactionnel avec une solution aqueuse saline saturée.

On préfère actuellement parmi les dérivés de formule III ceux dans lesquels X représente le cuivre, ou le chlorure ou le bromure de magnésium.

Selon le procédé de l'invention, la réaction des composés de formule générale III avec les composés de la formule générale IV s'effectue en présence d'un solvant organique inerte, de préférence un éther.

La température de la réaction dépend de plusieurs facteurs, notamment de la durée de la réaction, de la nature des catalyseurs, et du solvant utilisé. En général, ladite réaction s'effectue à une température comprise entre -20°C et +60°C, de telles valeurs limites ne devant cependant pas être considérées comme absolues.

Le catalyseur utilisé est un complexe organométallique d'un métal de transition et plus précisemment du nickel ou du palladium.

Le procédé décrit ci-dessus, objet de la présente invention, permet d'obtenir avec de bons rendements et en un nombre restreint d'étapes réactionnelles, de l'undécatriène-1,3E,5Z (I) ou des mélanges de I et II.

La nouveauté de l'invention réside dans le choix particulier de produits de départ simples qui permettent un procédé particulièrement avantageux. De plus, la réaction d'un organomagnésien vinylique avec un sulfure diénique est surprenante, ces deux produits étant inertes en l'absence d'un métal de transition.

La présente invention est illustrée de façon plus détaillée à l'aide des exemples ci-après :

**EXEMPLE 1** :

Undécatriène-1,3E,5Z (I) et undécatriène-1,3E,5E (II)

Mélange 50:50

A une solution de 50 millimoles de sulfure de butadiène-1E,3 et de butyle et de 3,25 millimoles de bromure de bis(triphenylphosphine) nickel $(2^{+})$ dans 200 ml de tetrahydrofuranne, ajouter 100 millimoles de bromure de heptène-1 yle magnésium (mélange de deux isomères Z et E dans un rapport pondéral 50 % et 50 %). Laisser la réaction se poursuivre sans refroidir le milieu réactionnel jusqu'à disparition totale des matières premières. Hydrolyser avec 100 ml d'une solution saturée de chlorure d'ammonium ou une autre solution aqueuse saline saturée. Agiter pendant une heure. Laisser ensuite décanter et récupérer la phase organique. Procéder à un deuxième lavage du milieu réactionnel

avec la solution aqueuse saline saturée et récupérer la phase organique. Sécher la phase organique sur sulfate de magnésium anhydre et éliminer le solvant en évaporant sous pression réduite (20mmHg) et à la température de 30°C. On obtient un mélange d'undécatriène-1,3E,5Z (I) et d'undécatriène-1,3E,5E (II). Distiller en présence d'un antioxydant sous pression réduite (0,05 mmHg) et à la température de 35° C pour obtenir un mélange pur de composés de formules I et II.

Le rendement de la réaction est de 50 %.

La composition du mélange a été déterminée par chromatographie en phase gazeuse, avec une colonne capillaire WCOT WAX 57 CB, de 10 mètres de long, avec programmation de la température 90°C - 200°C, 4°C/min. On obtient un mélange de I et II dans un rapport pondéral 1:1.

L'halogénure de heptène-1 yle magnésium utilisé comme produit de départ peut être synthétisé à partir de l'acide dibromo-2,3E octanoïque selon la méthode décrite par Norris (J.Org.Chem. (1959),25,1579). La synthèse de sulfure de butadiène-1E,3 et de butyle est connue (Everhardus et al.Rech.Trav.Chim. Pays-Bas 93,90,1974).

Les deux isomères I et II peuvent être obtenus à l'état pur, par séparation du mélange précédemment obtenu au moyen d'une chromatographie en phase gazeuse préparative. (phase stationnaire Carbowax à 50 %).

Les caractéristiques spectrales des deux isomères sont les suivantes :

A - Spectres de résonnance magnétique nucléaire du proton, enregistrés en 200 MHz en solution dans le deutero-chloroforme.

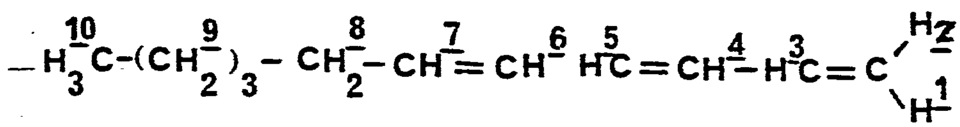

| Undécatriène -1,3E,5Z (I) | Undécatriène -1,3E,5E (II) | Attribution |
| --- | --- | --- |
| 0,88 (t) | 0,87 (t) | 3H10 |
| 1,30 (m) | 1,28 (m) | 6H9 |
| 2,18 (m) | 2,08 (m) | 2H8 |
| 5,06 | 5,05 | 1H1 |
| 5,19 | 5,18 | 1H2 |
| 5,47 | 5,74 | 1H7 |
| 6,02 | | 1H6 |
| 6,19 | 5,98-6,24(m) | 1H4 |
| 6,39 | | 1H3 |
| 6,51 | | 1H5 |

B - Spectres de l'infra-rouge, en solution dans le tetrachlorure de carbone.

Undécatriène-1,3E,5Z ($cm^{-1}$) : 3100, 3020, 1625, 1580, 900, 1000, 940, 750, 720.

Undécatriène-1,3E,5E ($cm^{-1}$) : 3100, 3030, 1630, 1585, 900, 1000, 975, 720.

C - Spectre de masse, enregistré à 70 eV, en impact éléctronique, après séparation chromatographique :

Undécatriène-1,3E,5Z (m/z) : 150($M^+$26%) 107(2%) 93(22%) 91(30%) 80(72%) 79(100%) 77(41%) 67(14%) 53(5%) 41(25 %).

Undécatriène-1,3E,5E (m/z) : 150($M^+$25%) 107(1%) 93(22%) 91(30%) 80(63%) 79(100%) 77(41%) 67(11%) 53(4%) 41(23%).

**EXEMPLE 2 :**

Undécatriène-1,3E,5Z (I) et undécatriène-1,3E,5E (II)
Mélange 70:30

A une solution de 50 millimoles de sulfure de butadiène-1E,3 et d'éthyle et de 3,25 millimoles de bromure de bis(triphenylphosphine) nickel ($2^+$) dans 200 ml de tetrahydrofuranne, ajouter 100 millimoles de bromure d' heptène-1 yle magnésium (mélange de deux isomères Z et E dans un rapport pondéral 70% et 30 %). Laisser la réaction se poursuivre sans refroidir pendant environ 3 heures. Hydrolyser avec 100 ml d'une solution saturée de chlorure d'ammonium en refroidissant avec un bain de glace. Agiter pendant une heure, décanter et

récupérer la phase organique. Procéder à un deuxième lavage du milieu réactionnel sous les conditions décrites ci-dessus.

Après séparation des deux phases, sécher la solution organique sur sulfate de magnésium anhydre et éliminer le solvant sous les conditions décrites à l'Exemple I. On obtient un mélange d'undécatriène-1,3E,5Z (I) et d'undécatriène-1,3E,5E (II) dans un rapport pondéral 7:3.

La constitution du mélange a été déterminée par chromatographie en phase gazeuse sous les conditions décrites à l'Exemple 1.

Le rendement global de la réaction est d'environ 48 %.

**EXEMPLE 3 :**
Undécatriène-1,3E,5Z (I) et undécatriène-1,3E,5E (II)
Mélange 60:40

A une solution de 50 millimoles de chloro-1 butadiène-1E,3 dans 200 ml de tetrahydrofuranne, ajouter sous azote, en refroidissant, 1,3 millimoles de bromure de bis(triphenyl-phosphine) nickel ($2^+$) et 100 millimoles de bromure de heptène-1 yle magnésium (mélange de deux isomères Z et E dans un rapport pondéral 6:4) en solution dans le tetrahydrofuranne. Chauffer à 35°C pendant environ 1 heure et hydrolyser ensuite le milieu réactionnel avec une solution saturée de chlorure d'ammonium. Décanter. Laver la phase organique par 2 fois 25 ml de la solution aqueuse saturée. Sécher la phase organique sur sulfate de magnésium anhydre. Eliminer le

solvant en évaporant sous pression réduite (20 mm Hg) et à la température de 30°C. On obtient un mélange d'undécatriène-1,3E,5Z (I) et d'undécatriène-1,3E,5E (II). Distiller sous pression réduite (0,02 mm Hg) et à la température de 30°C pour obtenir un mélange pur de deux isomères dans un rapport pondéral 6:4.

Le rendement global de la réaction est de 54 %.

La composition du mélange a été vérifiée par chromatographie en phase gazeuse avec colonne capillaire sous les conditions décrites à l'Exemple 1.

Le chloro-1 butadiène-1E,3 utilisé comme produit de départ est synthétisé selon la méthode décrite par Klebauskii (J.Gen.Chem. (1947),17,235).

**EXEMPLE 4 :**
Undécatriène-1,3E,5Z (I)

A une solution de 60 millimoles d' heptène-1Z yle cuivre dans 100 ml d'un mélange de tetrahydrofuranne et de diéthyle oxyde (75:25) ajouter en refroidissant 1,8 millimoles de bromure de bis(triphenylphosphine) nickel (O) et ensuite 50 millimoles de chloro-1 butadiène-1E,3 en solution dans 50 ml de tetrahydrofuranne. Laisser la réaction se poursuivre pendant 30 minutes, en agitant sans refroidir. Hydrolyser ensuite le milieu réactionnel avec 70 ml d'une solution aqueuse saturée de chlorure d'ammonium. Eliminer les sels minéraux formés, et ajouter 100 ml d'éther éthylique. La phase organique est lavée une fois avec la solution aqueuse saturée de chlorure d'ammonium et séchée ensuite sur sulfate

de magnésium anhydre. Les solvants organiques sont évaporés et le résidu distillé sous pression réduite (0,01 mm Hg) à la température de 26°C.

Les caractéristiques spectrales du produit ainsi obtenu sont identiques à celles décrites à l'Exemple 1 pour l'undécatriène-1,3E,5Z (I) (pureté 91 %).

Le rendement de la réaction est de 65 %.

L'heptène-1Z yle cuivre est synthétisé selon la méthode d'Alexakis (J.Organomet.Chem. (1979),177 293-298).

REVENDICATIONS

1) Procédé de préparation de l'undécatriène-1,3E,5Z pur (ou en mélange avec l'undécatriène-1,3E,5E) caractérisé en ce que l'on fait réagir un composé organométallique d'heptène-1Z yle pur (ou en mélange avec le composé organométallique correspondant à l'isomère 1E) de formule :

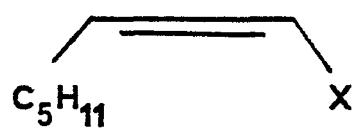

dans laquelle X repésente un groupe partant choisi parmi les métaux de transition, ou les halogènures de magnésium, avec un dérivé du butadiène-1E,3 de formule :

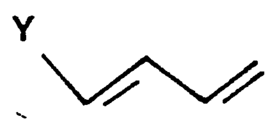

dans laquelle Y représente un groupe partant choisi parmi les atomes d'halogène ou les radicaux alkylthio de 1 à 4 atomes de carbone à une température comprise entre -20°C et +60°C en présence d'un catalyseur métallique dans un solvant organique, et on hydrolyse le milieu réactionnel, avec une solution aqueuse saline saturée.

2) Procédé selon la revendication 1, dans laquelle X représente un atome de cuivre, ou le chlorure ou le bromure de magnésium.

3) Procédé selon la revendication 1, caractérisé en ce que le catalyseur utilisé est un complexe organométallique d'un métal de transition.

4) Procédé selon la revendication 3 dans lequel le métal de transition est le nickel ou le palladium.

5) Procédé selon l'une des revendications 3 et 4, caractérisé en ce que le catalyseur est le bromure de bis(triphenylphosphine) nickel ($2^+$), ou le bromure de bis(triphenylphosphine) nickel(O).

6) Procédé selon la revendication 1, caractérisé en ce que la réaction s'effectue à une température comprise entre 0°C et +40°C.

0203615

Office européen des brevets

# RAPPORT DE RECHERCHE EUROPEENNE

Numero de la demande

EP 86 10 7380

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int Cl 4) |
|---|---|---|---|
| A | TETRAHEDRON LETTERS, no. 1, 1979, pages 43-46, Pergamon Press Ltd., GB; H. OKAMURA et al.: "Nickel-phosphine complex catalyzed coupling reaction of Grignard reagents with alkenyl or aryl sulfides"<br>----- | | C 07 C 11/21<br>C 07 C 1/30 |

| DOMAINES TECHNIQUES RECHERCHES (Int. Cl.4) |
|---|
| C 07 C 11/00<br>C 07 C 1/00 |

Le présent rapport de recherche a été établi pour toutes les revendications

| Lieu de la recherche | Date d'achèvement de la recherche | Examinateur |
|---|---|---|
| LA HAYE | 28-08-1986 | VAN GEYT J.J.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82